(19) Europäisches Patentamt
European Patent Office
Office européen des brevets

(11) Publication number: 0 292 101 A2

# (12) EUROPEAN PATENT APPLICATION

(21) Application number: 88302613.0

(22) Date of filing: 24.03.88

(51) Int. Cl.[4]: C07H 19/06 , C07H 15/04 , C07H 17/02

The title of the invention has been amended (Guidelines for Examination in the EPO, A-III, 7.3).

(30) Priority: 25.03.87 GB 8707101
23.05.87 GB 8712299

(43) Date of publication of application: 23.11.88 Bulletin 88/47

(84) Designated Contracting States: AT BE CH DE ES FR GB GR IT LI LU NL SE

(71) Applicant: THE WELLCOME FOUNDATION LIMITED
183-193 Euston Road
London NW1 2BP(GB)

(72) Inventor: Hurford, John Robert
The Wellcome Foundation Limited Temple Hill
Dartford Kent(GB)

(74) Representative: Garrett, Michael et al
The Wellcome Research Laboratories Group Patents and Agreements Langley Court
Beckenham Kent BR3 3BS(GB)

## (54) Process for the preparation of 3'-azido-3'-deoxythymidine and intermediates.

(57) This invention relates to a new synthetic process for the manufacture of zidovudine from the starting material D-xylose involving:

i) Conversion of D-xylose to a 2',3',5'-protected derivative of 1-(β-D-xylofuranosyl)thymine;
ii) 2'-Deoxygenation of the xylofuranosyl thymine; and
iii) 3'-Azidation of the 2'-deoxy compound.

EP 0 292 101 A2

## Chemical Process

The present invention relates to a novel process for the preparation of 3′-azido-3′-deoxythymidine and to intermediates of use in such a process.

The preparation of 3′azido-3′-deoxythymidine was first described by J.P. Horwitz et al., J. Org. Chem., 29, 2076-2078, 1964, and later for example by R. P. Glinski et al., Org. Chem, 38, 4299 - 4305, 1973 and T-S Lin et al., J. Med. Chem., 21 (1) 109-112 (1978). More recently, 3′-azido-3′-deoxythymidine has been discovered to have a potent antiviral activity against the human immunodeficiency virus (HIV), and has been reported to be of potential value for the therapeutic treatment of acquired immune deficiency syndrome (AIDS) (R. Yarchoan et al., The Lancet, 1 (8481), 575-580, Mar 15, 1986). Following further extensive clinical investigation this compound has been found to be of therapeutic benefit in the treatment of AIDS and AIDS-related complex (ARC). 3′-Azido-3′-deoxythymidine (otherwise named 1-(3′-azido-2′,3′-dideoxy-β-D-erythropentofuranosyl)-thymine) has recently been given the approved name zidovudine.

In view of the demand for relatively large quantities of zidovudine to fulfil further clinical testing requirements and to meet demands for the compound, considerable effort has been devoted towards ensuring adequate supplies of the compound on an industrial scale. A major difficulty that has been encountered in the commercial manufacture of zidovudine is that previously reported methods for the preparation of zidovudine (on a laboratory scale) have generally involved the use of thymidine as a starting material or intermediate. However, thymidine is a relatively expensive material and its commercial availability is relatively limited, which consequently limits the supply of zidovudine when prepared by previously reported methods.

We have therefore investigated alternative routes of synthesis for zidovudine starting from other, less expensive and more readily available, starting materials. As a result of considerable research and development, we have now discovered a synthetic route for zidovudine that uses, as a starting material, D-xylose which is a relatively inexpensive and readily commercially available starting material, thus avoiding the use of thymidine. The new synthetic route is characterised by a sequence of key steps involving certain novel intermediates in particular novel 2′-halonucleoside intermediates described in more detail below. These intermediates may then be converted to zidovudine by a series of steps essentially involving 2′-dehalogenation; introduction of a leaving group at the 3′-position, and replacement of the said leaving group by an azido group.

The overall synthetic route from D-xylose to zidovudine described below indicates the steps by which the 2′,3′,5′-protected 1-thymidyl derivative may be obtained from D-xylose in accordance with the invention and the steps by which this derivative may then be converted to zidovudine via the 2′halo derivative. The overall synthetic route will now be described in relation to the key steps referred to above which essentially involve (i) conversion of D-xylose to a 2′,3′,5′-protected derivative of 1-(β-D-xylofuranosyl) thymine (steps (a) to (d) below, (ii) formation of the 2′-halo derivative and subsequent reduction of the xylofuranosyl thymine (steps (e) to (h)) below, and (iii) 3′-azidation of the 2′-deoxy compound (steps (i) to (k)) below.

(i) Conversion of D-xylose to a 2′,3′,5′-blocked derivative of 1-(β-D-xylofuranosyl)thymine.

(a) Treating D-xylose of formula (I)

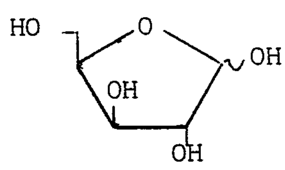

(I)

with a $C_{1-6}$ alkylating agent e.g. a $C_{1-6}$ alkanol, to form a compound of formula (II)

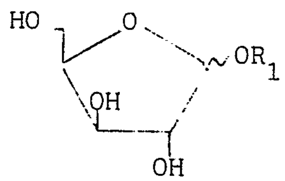

(II)

wherein $R_1$ is a $C_{1-6}$ alkyl group;

(b) treating a compound of formula (II) with hydroxy blocking agents to form a compound of formula (III)

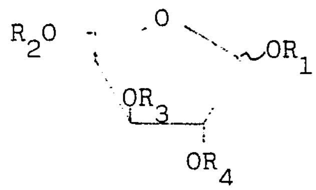

(III)

wherein $R_1$ is as hereinbefore defined and $R_2$, $R_3$ and $R_4$ each represent a hydroxy blocking group (for example an acyl group preferably an aromatic acyl group such as benzoyl or p-toluoyl)

(c) acylating a compound of formula (III) to form a compound of formula (IV)

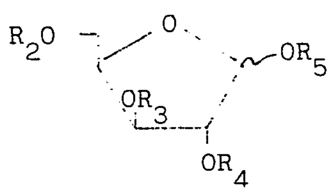

(IV)

wherein $R_2$, $R_3$ and $R_4$ are as hereinbefore defined and $R_5$ represents an acyl group, preferably acetyl.

(d) reacting a compound of formula (IV) with a thymine derivative of formula (V)

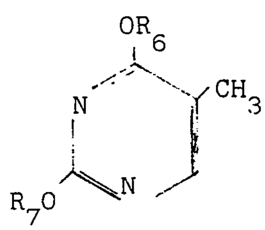

(V)

wherein $R_6$ and $R_7$ each represents a hydroxy blocking group (e.g. a trimethylsilyl group), with subsequent removal of the said $R_6$ and $R_7$ blocking groups to form a compound of formula (VI)

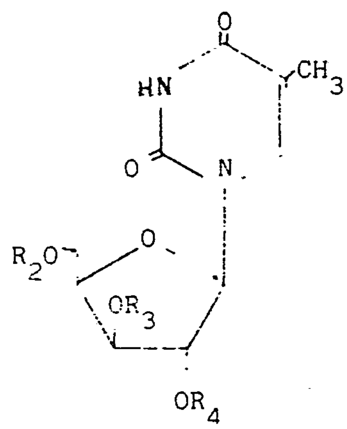

(VI)

wherein $R_2$, $R_3$ and $R_4$ are as hereinbefore defined.

With regard to step (a), the 1-hydroxy group of D-xylose is preferably blocked with a methyl group.

The reaction is effected using a $C_{1-6}$ alkylating agent e.g. a $C_{1-6}$ alkanol preferably methanol, advantageously in the presence of an acid e.g. hydrochloric acid.

With regard to step (b) the 2-, 3- and 5- hydroxy groups are for example blocked with acyl groups, preferably aromatic acyl groups e.g. benzoyl.In the case of acyl groups, blocking of the 2-, 3- and 5-hydroxy groups may be effected for example by treatment with an appropriate acyl halide (e.g. chloride) especially benzoyl chloride in the presence of a basic solvent such as pyridine. Suitable acyl blocking groups include pivaloyl and benzoyl, the latter being preferred. For pivaloyl blocking groups, the preferred reagent is trimethylacetyl chloride.

With regard to step (c), the selective acylation of the 1-hydroxy group may be effected for example using an acetic anhydride or halide e.g. chloride preferably in the presence of a strong acid such as sulphuric acid.

With regard to step (d), the blocking groups of the thymine derivative of formula (V) are preferably trimethylsilyl groups. Blocking of the hydroxy groups of thymine may be effected for example by refluxing the thymine in hexamethylenesilazane in the presence of ammonium sulphate or by refluxing in BSA or by heating the thymine with trimethylsilyl chloride in an aprotic solvent such as benzene in the presence of a base such as triethylamine.

The reaction of the compounds of formulae (IV) and (V) to form a compound of the formula (VI) is advantageously effected in the presence of a Lewis acid such as stannic chloride, preferably in an aprotic polar solvent such as acetonitrile or a halogenated hydrocarbon e.g. dichloroethane, generally at room temperature.

(ii) Formation of the 2′-halo derivative and subsequent 2′-deoxygenation of the xylofuranosyl thymine

(e) either treating a compound of formula (VI) with an agent or under conditions serving to effect deblocking of the 2′-hydroxy position of the xylofuranosyl moiety of the said compound and optionally to replace the said blocking groups $R_2$ and $R_3$ by a single group blocking both the 3′ and 5′ positions of the said moiety, to form a compound of formula (VII):-

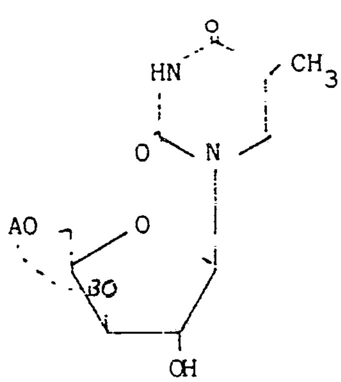

(VII)

in which A and B each represent a hydroxy blocking group or together form a single such group blocking the 3′ and 5′ positions;

(f) treating a compound of formula (VII) with an agent to introduce a leaving group at the 2′-hydroxy group of formula (VII) to form a compound of formula (VIII)

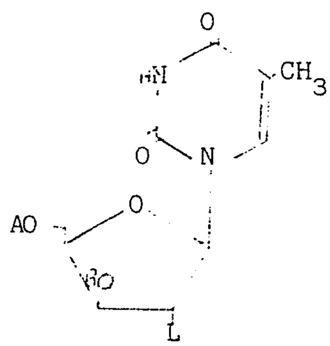

(VIII)

wherein A and B are as hereinbefore defined and L represents a leaving group such as a methanesulphonyloxy or a p-toluenesulphonyloxy group;

(g) treating a compound of formula (VIII) with an agent capable of replacing the leaving group L, with a reduceable halo group M, and optionally either removing the blocking groups A and B or replacing said blocking groups with alternative such groups, before or after treatment with the said agent, to form a compound of formula (IX)

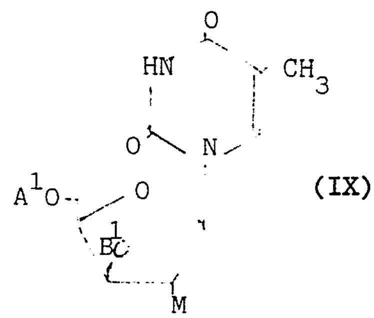

(IX)

wherein $A^1$ and $B^1$ are as hereinbefore defined or may represent hydrogen and M is a halo group; the said compound of formula (IX) being formed directly or optionally <u>via</u> an intermediate of formula (X)

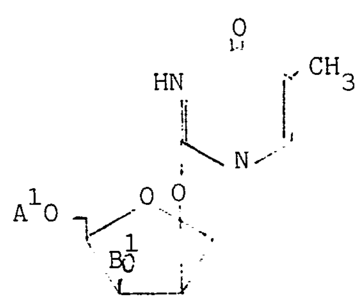

(X)

wherein $A^1$ and $B^1$ are as hereinbefore defined;

(h) reducing a compound of formula (IX) including removal of any combined groups blocking the 3′ and 5′ positions or any single 3′-blocking groups and optional removal of any 5′-hydroxy blocking groups or replacement of any such 5′-hydroxy blocking group by an alternative hydroxy blocking group, such removal or replacement being effected before or after said reduction, to form a compound of formula (XI)

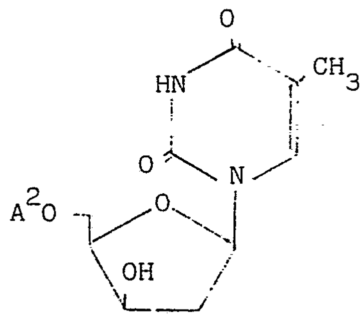

(XI)

wherein $A^2$ represents hydrogen or a hydroxy blocking group.

With regard to step (e), the selective removal of the 2'-hydroxy blocking group of the xylofuranosyl moiety may be effected for example when the blocking group is benzoyl, by using hydroxylaminium acetate preferably in the presence of a basic solvent such as pyridine at a temperature not greater than 20°C to form a compound of formula (VII). Alternatively, the compound of formula (VI) may be treated with for example methanolic ammonia to effect removal of the 2', 3' and 5'-blocking groups. The resulting compound may then be treated with an appropriate agent to effect introduction of a group blocking both the 3' and 5' positions. For example the 3'- and 5'- hydroxy groups may be blocked by an acetal or ketal group, e.g. an acetonide or benzylidene group, other such groups, including cyclic carbonate, and silyl ether groups. In the case of an acetonide group, blocking may be effected for example by treatment with dimethoxypropane, advantageously in the presence of a strong organic or inorganic acid such as hydrochloric acid, preferably in the presence of an apolar solvent such as acetone.

With regard to step (f) the leaving group L is advantageously an organosulphonyloxy e.g. an alkyl- or aryl-sulphonyloxy, for example methanesulphonyloxy, p-toluenesulphonyloxy or trifluoromethanesulphonyloxy group, the compound of formula (VII) being preferably treated with an appropriate acid halide for example mesyl chloride, preferably in the presence of a base for example an amine such as triethylamine and advantageously using for example a basic solvent such as pyridine.

With regard to step (g) the group M is advantageously a halo radical particularly bromo or iodo. These groups particularly in the case of a bromo group, are introduced using an appropriate agent for example a hydrohalide salt of a tertiary amine e.g. pyridine hydrobromide or with an alkali metal halide e.g. lithium bromide, in DMF.

When a compound of formula (VIII) is converted to a compound of formula (IX) via a novel 2,2'-anhydro derivative of formula (X), cyclisation of the compound of formula (VIII) where the 3' and 5' hydroxy groups together form a single blocking group may be effected in the presence of a strong base such as DBU or DBN, conveniently in a polar aprotic solvent such as DMF.

Cyclisation of a compound of formula (VIII) where the 3' and 5'-blocking groups are benzoyl may be effected by potassium carbonate in methanol and acetonitrile to form a novel 3',5'-hydroxy compound of formula (X).

A compound of formula (VII) may be converted to a compound of formula (X) without the need to isolate (VIII) by treating the compound of formula (VII) with an agent serving to introduce a carbonate group at the 2'-position (such as diphenyl carbonate), the resulting 2'-carbonate compound of formula (VIII) being converted in situ to the corresponding compound of formula (X) for example by treatment with a mild organic base such as sodium bicarbonate.

The compound of formula (X) is advantageously treated with an agent serving to introduce a reduceable group at the 2' position of the xylofuranosyl moiety. The reduceable group particularly in the case of a bromo group, may be introduced by treatment of the compound of formula (X) with an inorganic bromide, e.g. an alkali metal (such as sodium or lithium bromide, or an acyl bromide for example acetyl or benzoyl bromide, in which case any combined 3'- and 5'-blocking groups may be converted to single blocking groups. Individual acyl blocking groups may be introduced by reaction in an appropriate organic solvent, e.g. acetonitrile or dimethyl formamide but preferably acetic acid when acetyl bromide is used as described above.

Removal of blocking groups at the 3' and 5'-positions, may be effected for example by hydrolysis. In the case of acetal or ketal blocking groups, the hydrolysis is advantageously effected under acidic conditions e.g. in the presence of a strong acid such as hydrochloric acid.

In the case of ester blocking groups such as acyl or cyclic carbonate blocking groups, the hydrolysis is advantageously effected under acidic conditions, e.g. using hydrogen chloride in for example methanol,

while in the case of silyl ether groups, such hydrolysis may be effected for example by tetrabutyl ammonium fluoride.

In a preferred aspect of the invention a compound of formula (VII) may be converted directly to a compound of formula (IX) by a two stage reaction carried out in a single pot firstly using dialkylazodicarboxylate, a tri-alkyl or tri-aryl phosphine (e.g. $Ph_3P$) in an aprotic solvent (e.g. DMF) and secondly adding an alkali or alkaline earth metal bromide (e.g. LiBr) with a proton source (e.g. $KHSO_4.H_2O$)

In the case of a compound of formula (IX) where $A^1$ and $B^1$ are benzoyl groups, selective deblocking of the 3'-hydroxy group of the xylofuranosyl moiety may be effected using hydroxylaminium acetate preferably in the presence of a basic solvent such as pyridine at a temperature of greater than 20°C but not more than 70°C.

With regard to step h) the reduction of the compound of formula (IX) may be effected for example by catalytic hydrogenation advantageously in the presence of a palladium catalyst preferably at a temperature of 10°-30°C.

Where a compound of formula (XI) is formed in which $A^2$ represents hydrogen (i.e. a 5'-hydroxy compound), the said hydroxy group may be blocked for example by trityl or benzoyl groups introduced by an appropriate halide e.g. trityl chloride or benzoyl chloride, preferably in the presence of an aprotic base for example pyridine.

(iii) 3-Azidation of the 2'-deoxy compound of formula (XI)

(i) reacting a compound of formula (XI) with an agent serving to introduce a leaving group at the 3'-hydroxy position of the xylofuranosyl moiety of said compound to form a compound of formula (XII)

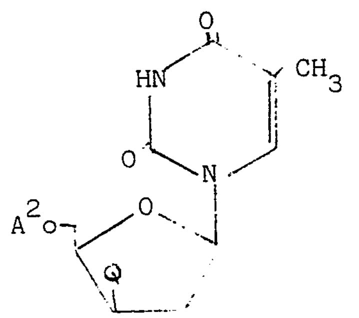

(XII)

wherein $A^2$ is as hereinbefore defined and Q is a leaving group e.g. a methanesulphonyloxy or a p-toluenesulphonyloxy group;

(j) reacting the compound of formula (XII) with an inorganic azide to form a compound of formula (XIII)

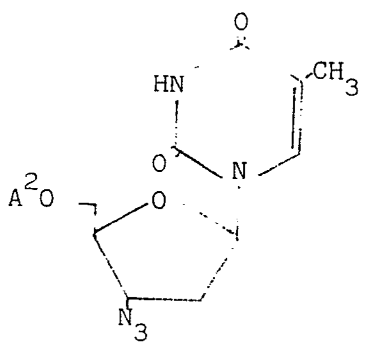

(XIII)

(wherein $A^2$ is as hereinbefore defined); and

(k) subsequently deblocking the compound of formula (XIII) to form zidovudine.

With regard to step (i) the agent serving to introduce the leaving group Q may be an appropriate acid halide for example mesyl chloride, preferably in the presence of a basic solvent such as pyridine and/or a base for example an amine such as triethylamine at a temperature of less than 20°C.

In step (j), the introduction of the 3'-azido group may be effected for example by an alkali metal azide, for example lithium or sodium azide preferably in the presence of an organic solvent such as dimethyl formamide. Finally with regard to step (k) the deblocking may be effected for example by treatment with a base, e.g. ammonia, sodium methoxide, sodium bicarbonate or sodium carbonate preferably in a solvent such as methanol.

The synthetic route described hereinbefore including the specific individual stages from D-xylose and the use of the specified intermediates provides a means for obtaining zidovudine in a highly pure form and a surprisingly good yield by convenient stages that can be readily developed for use on an industrial .-ale.

The development of the above stages and intermediates, certain of which are novel as referred to hereinafter, especially in combination to form the synthetic route as described above for the preparation of zidovudine, were the result of considerable research and investigation.

The present invention comprises each and every following feature:-

a) The novel compounds of formula (IX) and (X);

b) The novel compounds of formula (VIII) wherein A and B are each benzoyl;

c) The novel compounds of formula (XII) wherein $A^2$ is benzoyl;

d) The use of each and every compound of formula (I), (II), (III), (IV), (VI), (VII), (VIII), (IX), (X) in the synthesis of zidovudine particularly from the starting material D-xylose;

e) A compound of formula (I), (II), (III), (IV), (VI), (VII), (VIII), (IX), (X) for use in the synthesis of zidovudine particularly from the starting material D-xylose;

f) A process for the preparation of a compound of formula (IX) from a compound of formula (VIII) in accordance with stage (g) above;

g) A process for the preparation of a compound of formula (IX) directly from a compound of formula (VII) according to stage (g) above;

h) A process for the preparation of a compound of formula (XI) from a compound of formula (IX) in accordance with stage (g) above;

i) A process for the preparation of zidovudine from D-xylose involving stages a)-k) above, and each and every novel stage thereof.

The following Examples illustrate the present invention.

Example 1

Methyl-D-xylofuranoside (II)

(Baker, B.R., Shaub, R.E., and Williams, J.H., J. Amer. Chem. Soc. (1955), 77, 7).

D-xylose [132.0g; 0.88 mole] was stirred in a solution of hydrogen chloride dissolved in methanol [0.5% w/v; 2950 ml] at 20°C for 6 hours. The mixture was neutralised with methanolic sodium methoxide and most of the methanol evaporated at reduced pressure to afford the title compound as a yellow syrup containing traces of methanol and inorganic salts. Yield : 183.6g (127%).

To remove residual methanol, the syrup was co-evaporated with toluene [2 x 200 ml) and then from pyridine [2 x 200ml].

Example 2

Methyl 2,3,5-tri-O-benzoyl-D-xylofuranoside (III)

Methyl-D-xylofuranoside obtained as described in Example 1 [117.1g; eq. 0.56 mole D-xylose] was dissolved in chloroform [500 ml] and pyridine [180 ml] and cooled in an ice bath. With stirring, benzoyl chloride [248 ml; 2.13 moles] was introduced over a period of one hour, maintaining the temperature below 20°C. The mixture was allowed to stand at room temperature for 16 hours before the solvents were

removed at reduced pressure.

The resulting residue was dissolved in chloroform (600 ml) and washed successively with water (600 ml), saturated sodium hydrogen carbonate solution [3 x 300 ml] and finally with water [3 x 300 ml), before drying the organic extract over anhydrous magnesium sulphate.

Evaporation of the chloroform afforded the title compound as a red syrup. Yield: 312.3g (117.2% from D-xylose)

Example 3

1-O-Acetyl-2,3,5-tri-O-benzoyl-α-D-xylofuranose (IV)

(Nakayama, C and Saneyoshi, M. Nucleosides and Nucleotides, (1982), 1(2), 139.

Methyl 2,3,5-tri-O-benzoyl-D-xylofuranoside obtained as described in Example 2 [312.3g; e.g. 0.56 moles D-xylose] was dissolved in acetic acid (575 ml) and acetic anhydride [145 ml] with cooling to about 0°C. To this stirred solution over a period of 15 mins. concentrated sulphuric acid [86ml] was added keeping the temperature below 20°C. After standing the solution at 20°C for 16 hours, seed crystals of the α-acetate anomer were introduced which brought about crystallisation.

The crystallised product was collected by filtration and washed with methanol [2 x 100ml], followed by drying under vacuum. Yield (α-anomer] = 112g (39% based on D-xylose). m.p. 125.5-126°C.

Example 4

1-(2′,3′,5′-Tri-O-benzoyl-β-D-xylofuranosyl)thymine (VI)

(Fox, J.J., Yung, N, Davoll, J. and Brown, G. B., J. Amer Chem. Soc. (1956), 78, 2117.

Crude 1-O-acetyl-2,3,5-tri-O-benzoyl-α-D-xylofuranose obtained as described in Example 3 (55.0g; 0.109 moles) was dissolved along with 2,4-bis-trimethylsilylthymine [31.6g; 0.190 moles] in acetonitrile (300 ml), and cooled in an ice bath. Anhydrous stannic chloride [25.5 ml; 0.218 moles] was added in 3 portions (1 minute apart) to the stirred mixture, which was allowed to stand at 20°C for 16 hours.

Water (200 ml) was introduced to the yellow solution and the pH adjusted to 8 by the addition of solid sodium hydrogen carbonate (100g). The resulting slurry was filtered through a bed of Hyflo filter aid and the pad washed with hot chloroform [3 x 1000 ml].

The combined chloroform extract was washed with water [3 x 1000 ml], dried over anhydrous magnesium sulphate and evaporated to a residue which was dried under vacuum to yield the title compound. Yield: 49.8g (80.1%), as a pinkish solid. An NMR spectrum indicated an almost pure compound with peaks characteristic of the known β-anomer. Gosselin G., Bergogne M. de-rudder J., De-Clerg E. and Imbach J., J. Med. Chem. 1986 29 204.

A further quantity of the product[1.6 g; 2.1%] was obtained by re-extracting the Hyflo bed with chloroform.

Example 5

1-(3′,5′-Di-O-benzoyl-β-D-xylofuranosyl)thymine (VII)

(Gosselin, G., Bergogne, M.C., Imbach, J.L., Nucleosides Nucleotides (1984), 3(3), 265-75.

To a solution of 1-(2′,3′,5′-tri-O-benzoyl-β-D-xylofuranosyl) thymine obtained as described in Example 4 (100.0g; 0.175 mole) in pyridine (1000ml) was added hydroxylamine hydrochloride (48.8g; 0.702 mole) and anhydrous sodium acetate (57.6g; 0.702 mole) with vigorous stirring at 20°C. After 24 hours acetone (50ml) was added, most of the solvents were removed under vacuum and the resulting solids suspended in chloroform. The organic phase was stirred vigorously with iced hydrochloric acid (2N, 2000ml) for 1 hour to afford a heavy white precipitate which was collected on a Buchner head, washed with iced water (5 x 300 ml) and sucked as dry as possible.

The damp white solid (104g) was recrystallized from boiling SVM (120ml) to afford the title compound as fine white needle crystals on cooling to 4°C. Yield: 56.5g (69%), m.p. 155-6°C.

Example 6

1-(β-D-xylofuranosyl)thymine

Fox J.J., Yung N., Davoroll J. and Brown G.B., J. Amer. Chem. Soc., 1956, 78 21117. Gosselin G., Bergogne M., de-rudder J., De-Clerg F. and Imbach J., J. Med. Chem. 1986 29 204. 1-(2′,3′,5′-tri-O-benzoyl-β-xylofuranosyl)thymine as described in Example 4 (51.0g;0.089 moles], sodium hydrogen carbonate (5g) and methanol (400ml) were stirred together at reflux temperature for 1.5 hours (when all of the nucleosides had dissolved). The solution was filtered through a Florisil pad (60g) and then evaporated to a residue. Dissolution of the residue in water (250ml), followed by extraction with chloroform (4 x 50ml) removed methyl benzoate. The aqueous layer was evaporated to a residue which was dried under vacuum to afford a brown solid (24.9g).

To remove any remaining salts, the brown residue was redissolved in methanol (400ml) and passed though a second Florisil bed (50g) followed by evaporation and vacuum drying to yield an off-white material (23.1g).

The crude product was crystallised from a solution of boiling methanol (40ml), triturated with ethyl acetate (30ml) to faint turbidity. After cooling to 0°C with stirring, a further volume of ethyl acetate (180ml) was added and the slurry maintained at 4°C for 16 hours to yield the title compound. Yield : 13.9g (60.2%) as an off white crystalline solid, 1st crop m.p. 144-162°C. Using the standard procedures outlined above a further crop of material was obtained. Yield : 5.8g (25.1%), as an off-white solid. Total Yield: 19.7 g (85%)

The NMR spectra of first and second crops of compound were identical, both affording resonances in accord with the previously recorded β-anomer. Gosselin G., Bergogne M., de-rudder J., De-Clerg E. and Imbach J., J. Med. Chem., 1986 29 204.

Example 7

1-(3′,5′-O-Isopropylidene-β-D-xylofuranosyl)thymine (VII)

Crystalline 1-(β-D-xylofuranosyl)thymine obtained as described in Example 6 [5.2 g; 0.02 mole] was stirred vigorously at 20°C in a solution of acetone (20ml) and 2,2-dimethyloxypropane (20ml) containing concentrated hydrochloric acid (0.1 ml). After 2 hours the clear solution was evaporated to afford an off-white foam (6.8g) which crystallised from ethyl acetate (15ml) to afford the title compound as white crystals. Yield : 3.4g (57%), m.p. 179-82°C.

In a separate experiment 1-(β-D-xylofuranosyl)-thymine obtained as described in Example 6 [4.4g, 0.017 mole] was converted to the corresponding isopropylidene derivative as an off-white foam (5.0g, 98%), suitable for use in Example 9.

Example 8

1-(3',5'-Di-O-benzoyl-2'-O-mesyl-β-D-xylofuranosyl)thymine (VIII)

To a cooled stirred solution of 1-(3',5'-di-O-benzoyl-β-D-xylofuranosyl) thymine obtained as described in Example 5 (40g; 86mmole) dissolved in pyridine (100ml) and triethylamine (14.3ml; 102 mmole), mesyl chloride (12.6ml; 163mmole) was added slowly. The reaction mixture was allowed to gradually attain a temperature of 20°C and at 3 hours was quenched by pouring into iced, dilute acetic acid (20%, 600ml).

After stirring for 1 hour the pale yellow solid was collected on a Buchner head and washed with iced water (3 x 500ml) expelling from the solid as much water as possible. Recrystallization was achieved from hot SVM (400ml) and water (30ml) cooling slowly to 4°C to afford the title compound as fine white needle crystals. Yield : 325g (69.5%) m.p. 114-8°C.

Example 9

1-(3',5'-Isopropylidene-2'-O-mesyl-β-D-xylofuranosyl)thymine (VIII)

Crude 3',5'-O-isopropylidene derivative obtained as described in Example 7 [5.0g, 0.17 mole] in pyridine (33ml) was allowed to react with methanesulphonyl chloride [3ml, 0.037 mole] at 20°C for 1.5 hours and then at 40°C for 1 hour. Chromatography revealed one major new product.

Evaporation of the solvent gave a yellow residue which was dissolved in chloroform (60ml) and washed with ice code saturated sodium hydrogen carbonate solution (3 x 20ml) and water (1 x 40ml), finally drying the chloroform extract with anhydrous magnesium sulphate. Evaporation of the solvent provided the title compound as a yellow gum. Yield : 7.8g (123%)

Example 10

1-(3',5'-Di-O-benzoyl-2'-bromo-2'-deoxy-β-D-xylofuranosyl)thymine (IX)

A solution of 1-(3',5'-di-O-benzoyl-2'-O-mesyl-β-D-xylofuranosyl)thymine obtained as described in Example 8 (12g; 22mmole) in pyridine (80ml) was heated to reflux temperature in the presence of pyridine hydrobromide (7.2g; 45mmole). After 2 hours the reaction mixture was slowly poured into iced, dilute acetic acid (20% 500ml) to afford a fine white solid which was collected on a Buchner head and washed with iced water (3 x 500ml)to yield the title compound. Yield (crude): 7.7g (62%).

The off-white solid was recrystallized from hot SVM (100ml) and water (20ml) which upon cooling afforded fine white needle crystals. Yield (recryst.): 5.3g (45%) m.p. 119-23°C.

Example 11

1-(3',5'-Di-O-benzoyl-2'-bromo-2'-deoxy-β-D-xylofuranosyl)thymine (IX)

To a stirred solution of 1-(3',5'-di-O-benzoyl-β-D-xylofuranosyl) thymine obtained as described in Example 5 (10g, 21 mmole) and triphenylphosphine (9.56g, 36 mmole) in dimethylformamide (50ml), was added slowly (20 min.) diisopropyl azodicarboxylate (7.37g, 36mmole) dissolved in dimethylformamide (10ml), controlling the temperature to 20°C.

After 1 hour, lithium bromide (3.17g, 36mole), potassium hydrogen sulphate (4.96g, 36mmole), and water (1.0ml) were introduced to the solution with stirring at 90-100°C for 3 hours.

Upon cooling insoluble inorganic salts were removed by filtration followed by evaporation of most of the solvent. The yellow syrup was dissolved in ethanol (40ml) and stood at 4°C for 24 hours. The title compound was obtained as fine white crystals which were collected on a Buchner head, washed (ethanol) and dried. Yield : 6.1g (54%).

Example 12

1-(2,2'-Anhydro-3',5',O-isopropylidene-β-D-lyxofuranosyl)thymine (X)

The impure 2'-O-mesylate compound as obtained in Example 9 [7.8g; eg to 0.017 mole] was heated to 150°C in a solution of dimethylformamide and 1,8-diazabicyclo[5,4,0]-undec-7-ene [4.0 ml; 0.026 mole] for 8 hours. At this time chromatography revealed a single major new component and the absence of precursor.

High vacuum evaporation afforded a yellow syrup which crystallised from ethyl acetate (30ml) to afford fine needle crystals. After standing at 4°C for 2 hours the title compound was collected as white crystals. Yield : 3.9g (82%). An NMR spectrum of the title compound was in good agreement with the assigned structure.

For analytical purposes, the 3.9g of material was further purified by slurrying in hot methanol (15ml) and cooling to 0°C. After this procedure the weight of fine white needles was 2.9 g, m.p. 273-4°C.

Example 13

1-(3',5'-Di-O-acetyl-2'-bromo-2'-deoxy-β-D-xylofuranosyl)thymine (IX)

Purified 1-(2,2'-anhydro-3',5'-O-isopropylidene-β-D-lyxofuranosyl)thymine obtained as described in Example 12 [2.6g; 9.3 mmole] was heated and stirred at 100°C in a solution of acetic acid (25ml) and acetyl bromide [2.5ml; 33.8mmole]. After 45 mins the red solution was evaporated to a red glass (4.3 g). Silica-gel chromatography (70-230 mesh, 100g) of the product employing ethyl acetate and chloroform as eluent (1:1 v/v) afforded the title compound as a white foam. Yield : 3.4 g (90%). The product gave a sharp, well resolved NMR sepctrum in support of the proposed structure.

Example 14

1-(3',5'-Di-O-acetyl-2'-deoxy-β-D-xylofuranosyl)thymine (XI)

Chromatographically purified 1-(3',5'-di-O-acetyl-2'-bromo-2'-deoxy-β-D-xylofuranosyl)thymine obtained as described in Example 13 [0.5 g; 1.23 mmole] in 50% aqueous methanol (8 ml) containing sodium acetate trihydrate (0.3g) was hydrogenated over 5% palladium on barium sulphate (0.2g) at NTP.

Over a period of 30 minutes, the vigorously stirred solution absorbed about 50cc of hydrogen gas. Chromatographic examination of the reaction mixture revealed the absence of starting material and two major new products.

After filtering and evaporation, the residue was dissolved in water (7ml) and extracted with ethyl acetate (3 x 7ml). The combined extracts were dried over anhydrous sodium sulphate and evaporated to give the title compound as a colourless syrup. Yield : 0.32 g (79%) (crude).

An NMR spectrum of the crude syrup containing the above product revealed it to comprise of approximately 70% of the required title compound.

Example 15

1-(5'-O-Benzoyl-2'-bromo-2'-deoxy-β-D-xylofuranosyl)thymine

1-(3',5'-Di-O-benzoyl-2'-bromo-2'-deoxy-β-D-xylofuranosyl)thymine obtained as described in Example 10 (3.8g; 7.2mmol) was stirred in a pyridine solution (30ml) of hydroxylamine hydrochloride (1.82g; 26.2mmmole) and sodium acetate (2.14g; 26mmole) for 19 hours at 40°C. The mixture was poured slowly into iced, dilute acetic acid (20%, 200ml) the resulting off-white solid was collected on a Buchner head and washed with iced water (3 x 200ml), finally drying to constant weight, to yield the title compound. Yield (crude): 3.0g (98%). The material was recrystallised from hot SVM and water to afford fine, off white crystals. Yield (recryst.): 1.9g (62%), m.p. 114-7°C.

Example 16

1-(5'-O-Benzoyl-2'-deoxy-β-D-threo-pentofuranosyl thymine (XI)

(Binkley, R.W., Hehemann, D.G., Binkley, W.W., J. Org. Chem. (1978) 43 (13) 2573-6.

To a solution of 1-(5'-O-benzoyl-2'-bromo-2'deoxy-β-D-xylofuranosyl) thymine obtained as described in Example 15 (1.9g; 4.5mmole) in tetrahydrofuran (24ml) was added triethylamine (0.7ml, 5.0mmole) and 5% palladium on activated charcoal (0.9g) and the vigorously agitated mixture hydrogenated at NTP for 3 hours (120cc hydrogen absorbed). The solids were filtered and the filtrate evaporated to a pale yellow gum (1.5g) which was dissolved in acetone (4ml). The resulting small white precipitate (0.04g) was filtered and the filtrate triturated with cyclohexane to afford the title compound as white crystals. Yield : 0.9g (58%). An NMR spectrum of the product was in good agreement with the proposed structure.

Example 17

1-(2'-deoxy-β-D-threo-pentofuranosyl)thymine (XI)

1-(3',5'-di-O-acetyl-2'-deoxy-β-D-threo-pentofuranosyl)thymine obtained as described in Example 14 [0.3g; 0.92mmole] was warmed to 40°C in methanolic ammonia [4ml, 10% w/v] for 3 hours. Evaporation of the solvent afforded a white foam possessing the same chromatographic Rf as an authentic sample of the title compound. Yield : 0.25 g (104%) smelling faintly of acetamide.

Example 18

1-(2'-Deoxy-5'-O-trityl-β-D-threo-pentofuranosyl)thymine (XI)

1-(2'-deoxy-β-D-threo-pentofuranosyl)thymine obtained as described in Example 17 [0.25g; 1.0mmole] in pyridine (4ml) was treated with trityl chloride [0.38g; 1.4mmole] at 40°C for 5 h. Standard isolation procedures gave a sticky solid which was crystallised from ethyl acetate (4ml) to afford the title compound as off-white rossette crystals. Yield : 0.32g (67%).

The chromatographic and NMR data for the above porduct were in accord with an authentic sample.

Example 19

1-(2′-deoxy-3′-O-mesyl-5′-O-trityl-$\beta$-D-threo-pentofuranosyl)thymine (XI)

1-(2′-deoxy-$\beta$-D-threo-pentofuranosyl)thymine obtained as described in Example 17 [0.49g; 2,0mmole] in pyridine (8ml) was treated with trityl chloride [0.75g; 2.7mmole] at 40°C for 5 hr. The solution was cooled to 10-15°C and mesyl chloride [0.23; 3.0mmol] introduced, maintaining a 10-15°C temperature for 16 h. Standard isolation procedures afforded a pale yellow solid which was recrystallised from ethyl acetate to afford the title compound as off-white crystals. Yield : 0.87 g (75%).

Chromatographic and NMR data for the product were in full accord with an authentic sample.

Example 20

1-(5′-O-benzoyl-2′-deoxy-3′-mesyl-$\beta$-D-threo-pentofuranosyl)thymine (XII)

Crystalline 1-(5′-O-benzoyl-2′-deoxy-$\beta$-D-threo-pentofuranosyl) thymine obtained as described in Example 16 (0.8; 2.3mmole) in a chilled solution (0°C) of pyridine (3ml) and triethylamine (0.38ml; 2.7mmole) was treated with mesyl chloride (0.34ml; 4.3mmole). Over a period of 3 hours the mixture was allowed to attain 20°C before quenching in iced, dilute acetic acid (20%, 25ml). With stirring the resulting sticky solid became more friable and was collected on a Buchner head washed with water (3 x 100ml) and dried in air to yield the title compound. Yield (crude): 1.0g (102%) The above product was purified over silica gel (35g; 70-230 mesh) employing ethyl acetate as mobile phase to afford the title compound as a white foam. Yield (purified): 0.75g (76%).

An NMR spectrum of the product was in good agreement with the proposed structure.

Example 21

1-(3′-Azido-5′-O-benzoyl-2′,3′-dideoxy-$\beta$-D-erythro-pentofuranosyl)thymine (XIII)

1-(5′-O-benzoyl-2′-deoxy-3′-O-mesyl-$\beta$-D-threo-pentofuranosyl)thymine obtained as described in Example 20 (0.55g; 1.3mmole) was stirred with finely divided sodium azide (0.13g; 2.0mmole) in dimethylformamide (3ml) at a temperature of 90°-95°C (oil bath). The mixture was triturated with iced water (25ml) after 4 hours and the sticky white solid extracted into ethyl acetate (3 x 15ml). Combined organic layers were washed with water (2 x 15ml) and the solvent evaporated to afford the title compound as an off-white foam. Yield : 0.45g (94%).

Examination by tlc (ethyl acetate eluent) revealed the title compound to be a single spot.

Example 22

1-(3′-azido-2′,3′-dideoxy-5′-O-trityl-$\beta$-D-erythro-pentofuranosyl)thymine (XIII)

Recrsyallised 1-(2′-deoxy-3′-O-mesyl-5′-O-trityl-$\beta$-D-threo-pentofuranosyl)thymine obtained as described in Example 19 [0.80g; 1.4mmole] in the presence of sodium azide [0.10g; 1.54mmole] was stirred in a solution of DMF (6ml) at 95°C for 4.5 hours.

The product was isolated by firstly destroying excess azide ions with sodium nitrite [0.10g; 1.5mmole] dissolved in a solution of 20% aqueous acetic acid (2ml) with stirring for 1 hour at 20°C. Additional aqueous acetic acid (12ml) was added to precipitate the title compound which was isolated by filtration and drying. Yield : 0.6g (84%).

Example 23

3′-Azido-3′-deoxythymidine

1-(3′-Azido-5′-O-benzoyl-2′,3′-dideoxy-β-D-erythro-pentofuranosyl) thymine obtained as described as Example 21 (0.38g; 1.0mmole) was heated with sodium hydrogen carbonate (0.06g) in methanol and heated to reflux temperature for 3 hours. Water (10ml) was introduced and the solution evaporated at reduced pressure to a thin syrup. Further water (10ml) was introduced and the volume reduced to approximately (3ml) which upon cooling provided the title compound as fine white crystals. Yield : 0.16g (58%) m.p. 122-4°C; mixed m.p. with authentic 509U 122-4°C; m.p. of authentic 509U 123-5°C (All melting points were uncorrected).

Example 24

3′-Azido-3′-deoxythymidine

1-(3′-azido-2′,3′-dideoxy-5′-O-trityl-β-D-erythro-pentofuranosyl) thymine obtained as described in Example 22 [0.60g; 1.2mmole] was suspended in a solution of 50% aqueous methylated alcohol (5ml) adjusted to pH 2.0 with hydrochloric acid, and refluxed for 4 hours. On cooling to 20°C, triphenylmethanol crystallised out and was removed by filtration. The mother liquors were evaporated to remove most of the alcohol. On cooling to 10°C the title compound (14) crystallised from solution as white solid. Yield : 0.25g (76%).

The product was shown to be identical in every aspect with an authentic sample of the compound.

**Claims**

1. A novel compound of formula (A).

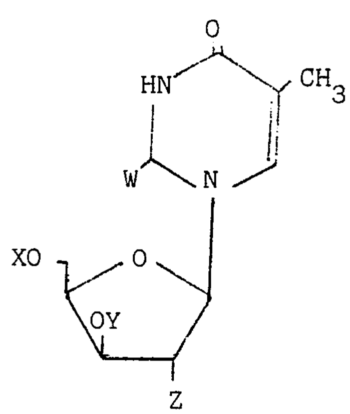

(A)

wherein X and Y each represent a hydroxy blocking group or together form a single such group blocking the 3′ and 5′ positions, W represents an oxo group and Z is a halo group; or W and Z together represent an -O- linkage between the 2 and 2′-positions of the compound of formula (A); or Z is a mesyl group, W is an oxo group and X and Y each represent a benzoyl blocking group; or Z represents hydrogen, W is an oxo group, Y is a mesyl group and X is a benzoyl blocking group.

2. A compound according to claim 1 wherein X and Y are hydroxy blocking groups and Z is bromo.

3. A compound according to claim 1 wherein X and Y together form a single blocking group and W and Z together represent an -O- linkage between the 2 and 2′-positions of the compound of formula (A)

4. A compound of formula (I)

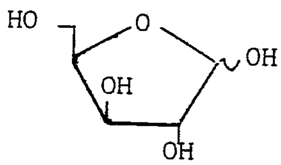

(I)

for use in the synthesis of zidovudine.

5. A compound of formula (II)

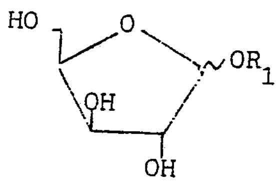

(II)

wherein $R^1$ is a $C_{1-6}$ alkyl group, for use in the synthesis of zidovudine

6. A compound of formula (III)

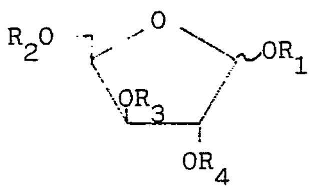

(III)

wherein $R^1$ is as defined in claim 5 and $R^2$, $R^3$ and $R^4$ each represent a hydroxy blocking group, for use in the synthesis of zidovudine.

7. A compound of formula (IV)

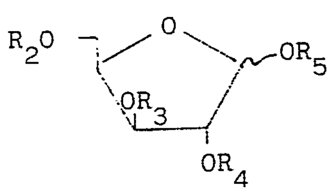

(IV)

wherein $R^2$, $R^3$ and $R^4$ are as defined in claim 6 and $R^5$ represents an acyl group, for use in the synthesis of zidovudine.

8. A compound of formula (VI)

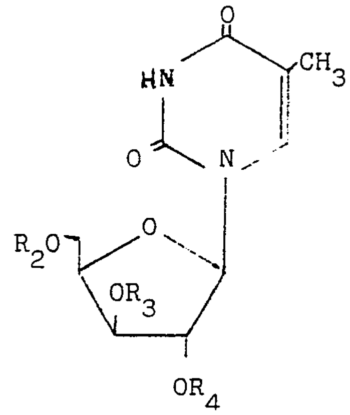

(VI)

wherein $R^2$, $R^3$ and $R^4$ are as defined in claim 6, for use in the synthesis of zidovudine.

9. A compound of formula (VII)

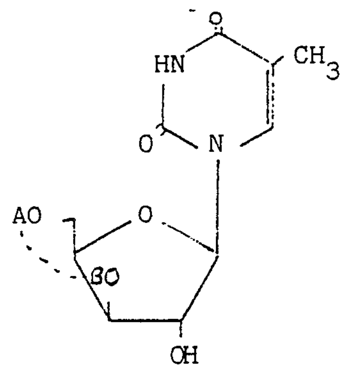

(VII)

wherein A and B each represent a hydroxy blocking group or together form a single such group blocking the 3′ and 5′ positions, for use in the synthesis of zidovudine.

10. A compound of formula (VIII)

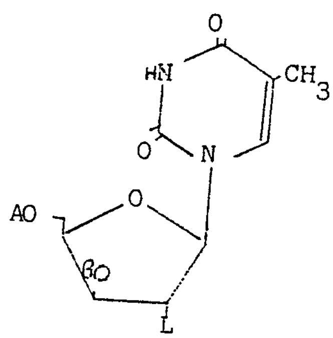

(VIII)

wherein A and B are as defined in claim 9 and L represents a leaving group, for use in the synthesis of zidovudine.

11. A compound of formula (IX)

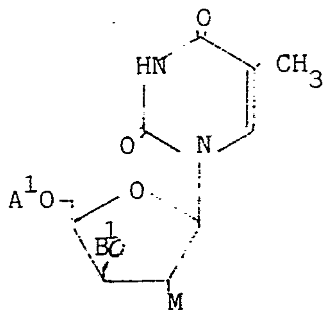

(IX)

wherein A¹ and B¹ are as defined in claim 9 or may represent hydrogen and M is a halo group, for use in the synthesis of zidovudine.

12. A compound of formula (X)

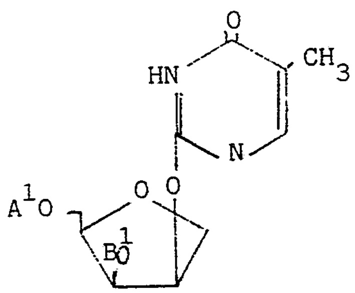

(X)

wherein A¹ and B¹ are as defined in claim 11, for use in the synthesis of zidovudine.

13. A process for the preparation of a compound of formula (IX)

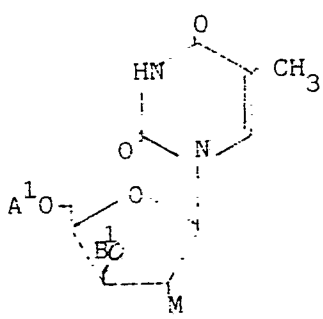

(IX)

wherein A¹ and B¹ are as defined in claim 9 or may represent hydrogen, and M is halo, which comprises treating a compound of formula (VIII)

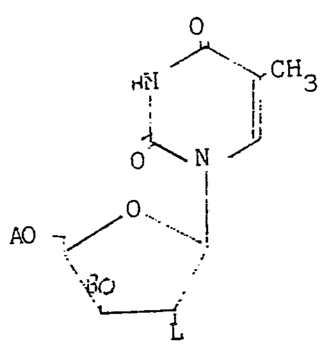

(VIII)

wherein A and B are as defined above and L represents a leaving group, with an agent capable of replacing the leaving group L, with a reduceable halo group M, and optionally either removing the blocking groups A and B or replacing said blocking groups with alternative such groups, before or after treatment with the said

agent.

14. A process for the preparation of a compound of formula (IX)

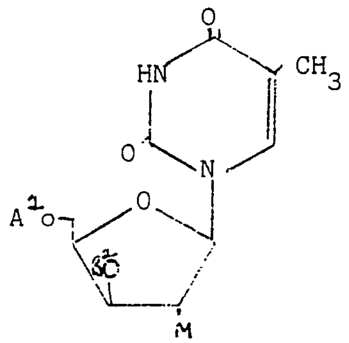

(IX)

wherein $A^1$ and $B^1$ are as defined in claim 13, which comprises treating a compound of formula (VII)

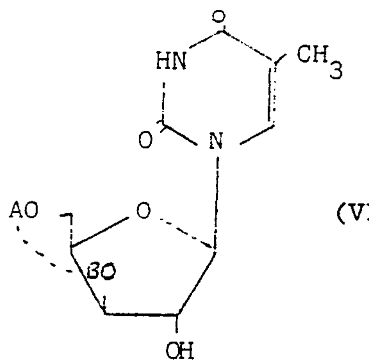

(VII)

in which A and B are as defined above, with dialkylazodicarboxylate, and a tri-alkyl or tri-aryl phosphine and subsequently with an alkali or an alkaline earth metal halide.

15. A process for the preparation of a compound of formula (XI)

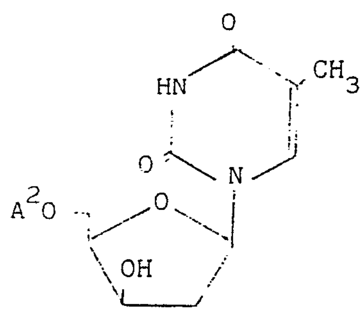

(XI)

wherein $A^2$ represents hydrogen or a hydroxy blocking group, which comprises reducing a compound of formula (IX) wherein $A^1$ and $B^1$ are as defined in claim 13 including removal of any combined groups blocking the 3′ and 5′ positions, removal of any single 3′-blocking groups and optional removal of any 5′-hydroxy blocking groups or replacement of any such 5′-hydroxy blocking groups by an alternative hydroxy blocking group, such removal or replacement being effected before or after said reduction.

16. A process for the preparation of zidovudine from D-xylose involving

(a) treating D-xylose of formula (I) as defined in claim (4) with a $C_{1-6}$ alkylating agent, to form a compound of formula (II) as defined in claim (5);

(b) treating a compound of formula (II) as defined in claim 5 with hydroxy blocking agents to form a compound of formula (III) as defined in claim 6.

(c) acylating a compound of formula (III) as defined in claim 6 to form a compound of formula (IV) as defined in claim 7;

(d) reacting a compound of formula (IV) as defined in claim 7 with a thymine derivative of formula (V)

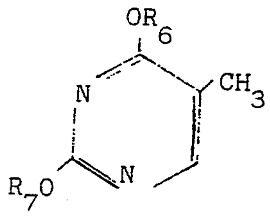

(V)

wherein $R^6$ and $R^7$ each represents a hydroxy blocking group, with subsequent removal of the said $R^6$ and $R^7$ blocking groups to form a compound of formula (VI) as defined in claim 8;

(e) either treating a compound of formula (VI) as defined in claim 8 with an agent or under conditions serving to effect deblocking of the 2′-hydroxy position of the xylofuranosyl moiety of the said compound and optionally replacing the said blocking groups $R^2$ and $R^3$ by a single group blocking both the 3′ and 5′ positions of the said moiety, to form a compound of formula (VII) as defined in claim 9.

(f) treating a compound of formula (VII) as defined in claim 9 with an agent to introduce a leaving group at the 2′-hydroxy group of formula (VII) to form a compound of formula (VIII) as defined in claim 10;

(g) treating a compound of formula (VIII) as defined in claim 10 with an agent capable of replacing the leaving group L with a reduceable halo group M, and optionally either removing the blocking groups A and B or replacing said blocking groups with alternative such groups, before or after treatment with the said agent, to form a compound of formula (IX) as defined in claim 11; the said compound of formula (IX) being formed directly or optionally via an intermediate of formula (X) as defined in claim 12;

(h) reducing a compound of formula (IX) as defined in claim 11 including removal of any combined groups blocking the 3′ and 5′ positions or any single 3′-blocking groups and optional removal of any 5′-hydroxy blocking groups or replacement of any such 5-hydroxy blocking groups by an alternative hydroxy blocking group, such removal or replacement being effected before or after said reduction, to form a compound of formula (XI)

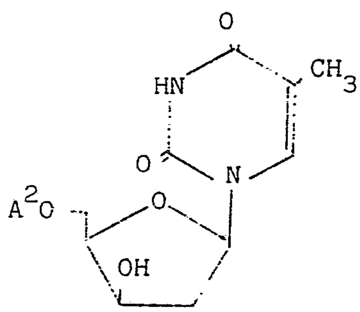

(XI)

wherein $A^2$ represents hydrogen or a hydroxy blocking group.

(i) reacting a compound of formula (XI) as defined above an agent serving to introduce a leaving group at the 3′-hydroxy position of the xylofuranosyl moiety of said compound to form a compound of formula (XII)

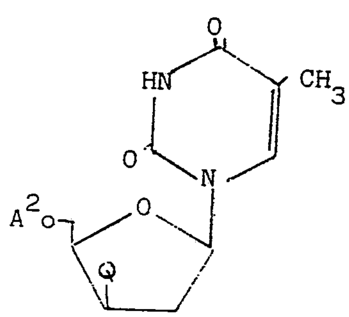

(XII)

wherein $A^2$ is as defined above and Q is a leaving group;

(j) reacting the compound of formula (XII) as defined above with an inorganic azide to form a compound of formula (XIII)

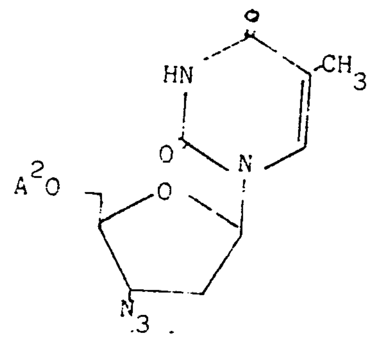

(XIII)

wherein $A^2$ is defined above; and

(k) subsequently deblocking the compound of formula (XIII) to form zidovudine.

17. A process for the preparation of zidovudine involving:

a) the reduction of a compound of formula (IX) as defined in claim 11 to give a compound of formula (XI) as defined in claim 15;

b) the introduction of a leaving group to a compound of formula (XI) as defined above to give a compound of formula (XII) as defined in claim 16(i); and

c) the subsequent azidation and deblocking of a compound of formula (XII) as defined above to give zidovudine.

18. A process for the prearation of zidovudine involving:

a) the azidation of a compound of formula (XII) as defined in claim 16(i) wherein $A^2$ is benzoyl; and

b) the subsequent removal of said benzoyl group to give zidovudine.

Claims for the following contracting States: GR and ES

1. A process for the preparation of a compound of formula (A)

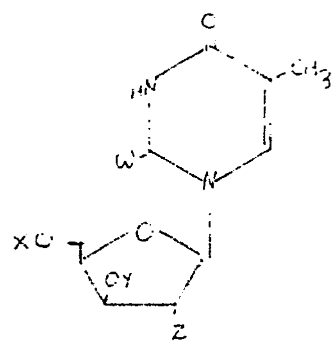

(A)

wherein X and Y each represent a hydroxy blocking group or together form a single such group blocking the 3′ and 5′ positions, W represents an oxo group and Z is halo; or W and Z together represent an -O- linkage between the 2- and 2′-positions of the compound of formula (A); or Z is a mesyl group, W is an oxo group and X and Y each represent a benzoyl blocking group; or Z represents hydrogen, W is an oxo group, Y is a mesyl group and X is a benzoyl blocking group;

Comprising either:

a) where Z is halo, halogenating a compound of formula

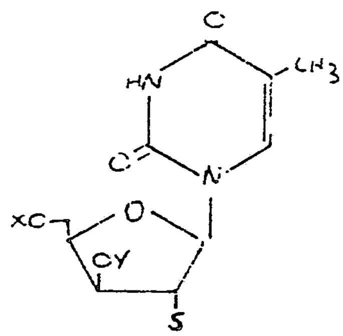

wherein X and Y are as hereinbefore defined and S represents a hydroxy or leaving group; or

b) where W and Z together form an -O- linkage as defined above, cyclising a compound of formula,

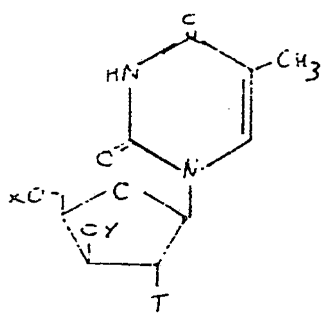

wherein X and Y are as hereinbefore defined and T represents a leaving group; or

c) where Z is a mesyl group and X and Y represent benzoyl blocking groups and W is oxo, mesylating a compound of formula

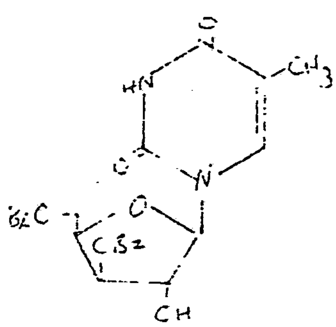

or

d) where Z is hydrogen, Y is mesyl, X is benzoyl and W is oxo, mesylating a compound of formula

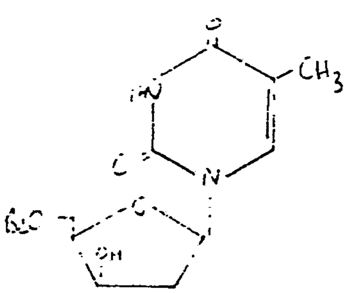

2. A process for the preparation according to claim 1 of a compound wherein X and Y are hydroxy blocking groups and Z is bromo.

3. A process for the preparation according to claim 1 of a compound wherein X and Y together form a single blocking group and W and Z together represent an -O- linkage between the 2- and 2'-positions of the compound of formula (A)

4. Use of a compound of formula (I)

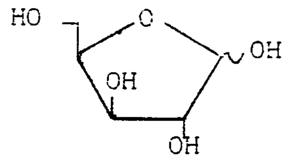

(I)

in the synthesis of zidovudine.

5. Use of a compound of formula (II)

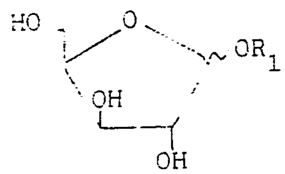

(II)

wherein $R^1$ is a $C_{1-6}$ alkyl group, in the synthesis of zidovudine.

6. Use of a compound of formula (III)

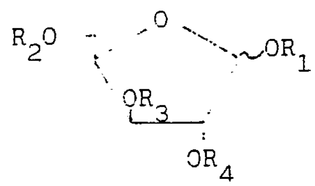

(III)

wherein $R^1$ is as defined in claim 5 and $R^2$, $R^3$ and $R^4$ each represent a hydroxy blocking group, in the synthesis of zidovudine.

7. Use of a compound of formula (IV)

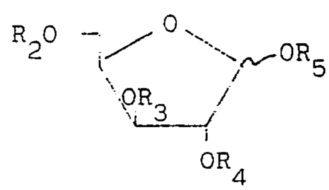

(IV)

wherein $R^2$, $R^3$ and $R^4$ are as defined in claim 6 and $R^5$ represents an acyl group, in the synthesis of zidovudine

8. Use of a compound of formula (VI)

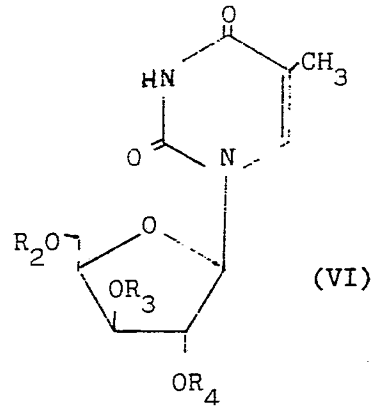

(VI)

wherein $R^2$, $R^3$ and $R^4$ are as defined in claim 6, in the synthesis of zidovudine.

9. Use of a compound of formula (VII)

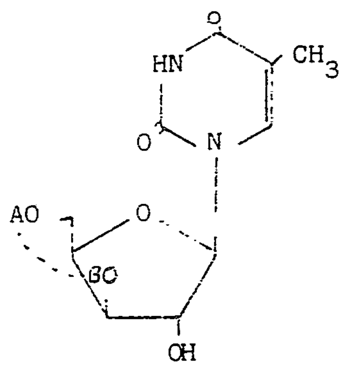

(VII)

wherein A and B each represent a hydroxy blocking group or together form a single such group blocking the 3′ and 5′ positions, in the synthesis of zidovudine.

10. Use of a compound of formula (VIII)

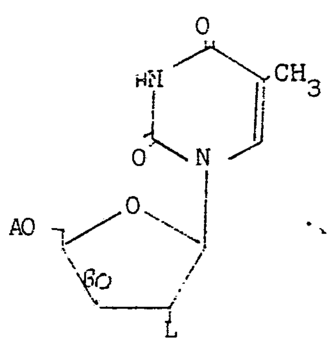

(VIII)

wherein A and B are as defined in claim 9 and L represents a leaving group, in the synthesis of zidovudine.

11. Use of a compound of formula (IX)

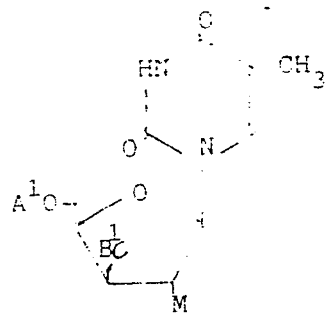

(IX)

wherein $A^1$ and $B^1$ are as defined in claim 9 or may represent hydrogen, and M is a halo group, in the synthesis of zidovudine.

12. Use of a compound of formula (X)

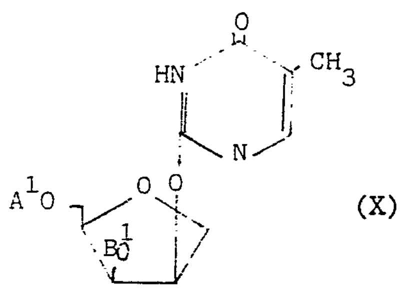

(X)

wherein $A^1$ and $B^1$ are as defined in claim 11, in the synthesis of zidovudine.

13. A process for the preparation of a compound of formula (IX)

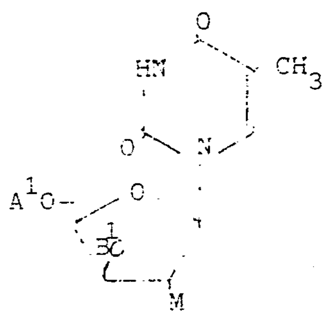

(IX)

wherein $A^1$ and $B^1$ are as defined in claim 9 or may represent hydrogen, and M is halo, which comprises treating a compound of formula (VIII)

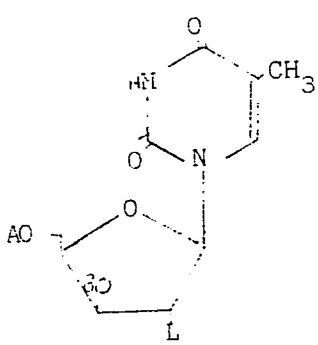

(VIII)

wherein A and B are as defined above and L represents a leaving group, with an agent capable of replacing the leaving group L, with a reduceable halo group M, and optionally either removing the blocking groups A and B or replacing said blocking groups with alternative such groups, before or after treatment with the said

agent.

14. A process for the preparation of a compound of formula (IX)

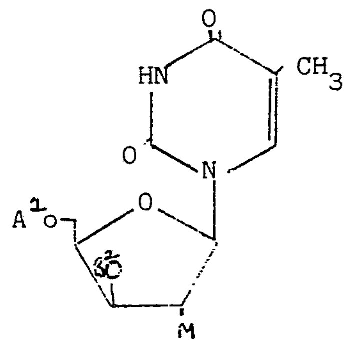

(IX)

wherein $A^1$ and $B^1$ are as defined in claim 13, which comprises treating a compound of formula (VII)

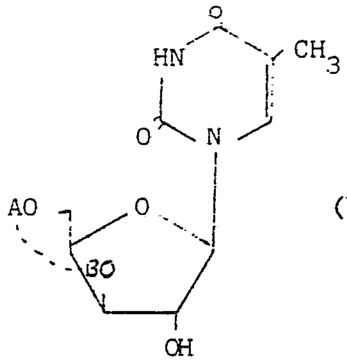

(VII)

in which A and B are as defined above, with dialkylazodicarboxylate, and a tri-alkyl or tri-aryl phosphine and subsequently with an alkali or an alkaline earth metal halide.

15. A process for the preparation of a compound of formula (XI)

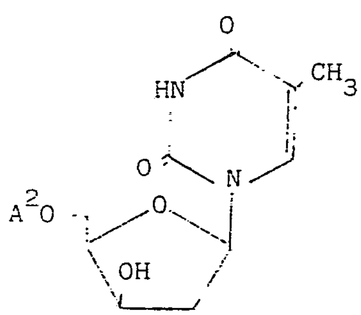

(XI)

wherein $A^2$ represents hydrogen or a hydroxy blocking group, which comprises reducing a compound of formula (IX) wherein $A^1$ and $B^1$ are as defined in claim 13 including removal of any combined groups blocking the 3′ and 5′ positions, removal of any single 3′-blocking groups and optional removal of any 5′-hydroxy blocking groups or replacement of any such 5′-hydroxy blocking groups by an alternative hydroxy blocking group, such removal or replacement being effected before or after said reduction.

16. A process for the preparation of zidovudine from D-xylose involving

(a) treating D-xylose of formula (I) as defined in claim (4) with a $C_{1-6}$ alkylating agent, to form a compound of formula (II) as defined in claim (5);

(b) treating a compound of formula (II) as defined in claim 5 with hydroxy blocking agents to form a compound of formula (III) as defined in claim 6;

(c) acylating a compound of formula (III) as defined in claim 6 to form a compound of formula (IV) as defined in claim 7;

(d) reacting a compound of formula (IV) as defined in claim 7 with a thymine derivative of formula (V)

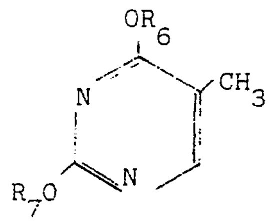

(V)

wherein $R^6$ and $R^7$ each represents a hydroxy blocking group, with subsequent removal of the said $R^6$ and $R^7$ blocking groups to form a compound of formula (VI) as defined in claim 8;

(e) either treating a compound of formula (VI) as defined in claim 8 with an agent or under conditions serving to effect deblocking of the 2′-hydroxy position of the xylofuranosyl moiety of the said compound and optionally replacing the said blocking groups $R^2$ and $R^3$ by a single group blocking both the 3′ and 5′ positions of the said moiety, to form a compound of formula (VII) as defined in claim 9.

(f) treating a compound of formula (VII) as defined in claim 9 with an agent to introduce a leaving group at the 2′-hydroxy group of formula (VII) to form a compound of formula (VIII) as defined in claim 10;

(g) treating a compound of formula (VIII) as defined in claim 10 with an agent capable of replacing the leaving group L with a reduceable halo group M, and optionally either removing the blocking groups A and B or replacing said blocking groups with alternative such groups, before or after treatment with the said agent, to form a compound of formula (IX) as defined in claim 11; the said compound of formula (IX) being formed directly or optionally via an intermediate of formula (X) as defined in claim 12;

(h) reducing a compound of formula (IX) as defined in claim 11 including removal of any combined groups blocking the 3′ and 5′ positions or any single 3′-blocking groups and optional removal of any 5′-hydroxy blocking groups or replacement of any such 5-hydroxy blocking groups by an alternative hydroxy blocking group, such removal or replacement being effected before or after said reduction, to form a compound of formula (XI)

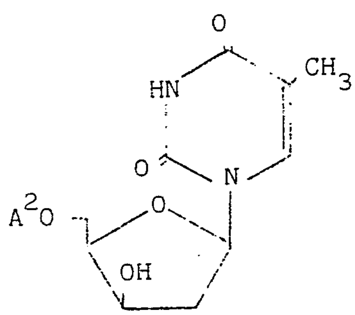

(XI)

wherein $A^2$ represents hydrogen or a hydroxy blocking group.

(i) reacting a compound of formula (XI) as defined above with an agent serving to introduce a leaving group at the 3′-hydroxy position of the xylofuranosyl moiety of said compound to form a compound of formula (XII)

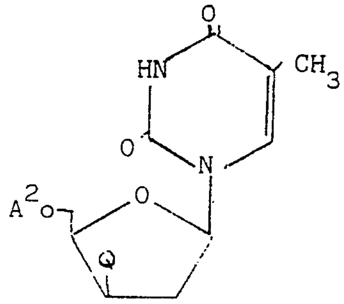

(XII)

wherein $A^2$ is as defined above and Q is a leaving group;

(j) reacting the compound of formula (XII) as defined above with an inorganic azide to form a compound of formula (XIII)

(XIII)

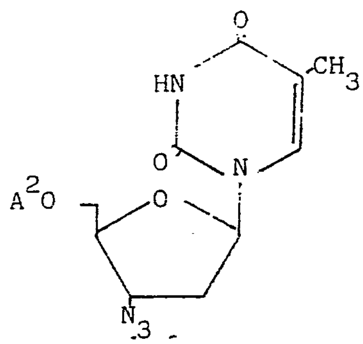

wherein $A^2$ is defined above; and

(k) subsequently deblocking the compound of formula (XIII) to form zidovudine.

17. A process for the preparation of zidovudine involving:

a) the reduction of a compound of formula (IX) as defined in claim 11 to give a compound of formula (XI) as defined in claim 15;

b) the introduction of a leaving group to a compound of formula (XI) as defined above to give a compound of formula (XII) as defined in claim 16(i); and

c) the subsequent azidation and deblocking of a compound of formula (XII) as defined above to give zidovudine.

18. A process for the preparation of zidovudine involving:

a) the azidation of a compound of formula (XII) as defined in claim 16(i) wherein $A^2$ is benzoyl; and

b) the subsequent removal of said benzoyl group to give zidovudine.